# EUROPEAN PATENT SPECIFICATION

(11) **EP 1 049 694 B1**
(45) Date of publication and mention of the grant of the patent: **15.05.2002**
(21) Application number: 99934200.9
(22) Date of filing: 01.02.1999
(51) Int. Cl.: C07D 417/12, C07D 403/12, C07D 413/12, C07D 473/34, C07D 487/04, C07D 417/04, A61K 31/505

(54) **DERIVATIVES OF BENZOXADIAZOLES, BENZOTHIADIAZOLES, BENZOTRIAZOLES AND QUINOXALINES**
DERIVATE VON BENZOXADIAZOL, BENZOTHIADIAZOL, BENZOTRIAZOL UND CHINOXALIN
DERIVES DE BENZOXADIAZOLES, BENZOTHIADIAZOLES, BENZOTRIAZOLES ET QUINOXALINES

(30) Priority: 03.02.1998 GB 9802251
(43) Date of publication of application: 08.11.2000
(73) Proprietor: Novartis AG, 4056 Basel (CH); Novartis-Erfindungen Verwaltungsgesellschaft m.b.H., 1235 Wien (AT)
(72) Inventor: NEUMANN, Bernhard, Peter, 3013 Bern (CH)
(74) Representative: Becker, Konrad
(86) International application number: EP9900622
(87) International publication number: WO9940089

(56) References cited:
- EP-A- 0 602 851
- EP-A- 0 640 595
- GB-A- 2 093 450

## Description

The present invention relates to novel derivatives of benzoxadiazoles, benzothiadiazoles, benzotriazoles and quinoxalines, their preparation, their use as pharmaceuticals and pharmaceutical compositions containing them

More particularly the invention provides a compound of formula I 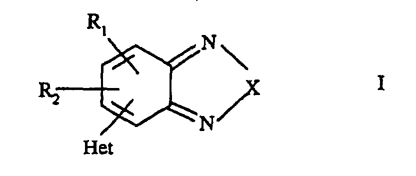 wherein
- X: is O, S, N-CH₃, CH=CH or CAlk = CAlk. where the Alk independently are (C₁₋₄)alkyl,
- R₁ and R₂: independently, are hydrogen, halogen, (C₁₋₄)akyl, (C₁₋₄)alkoxy or trifluoromethyl, and
- Het: is a radical having one of the formulae (a) to (p) below: 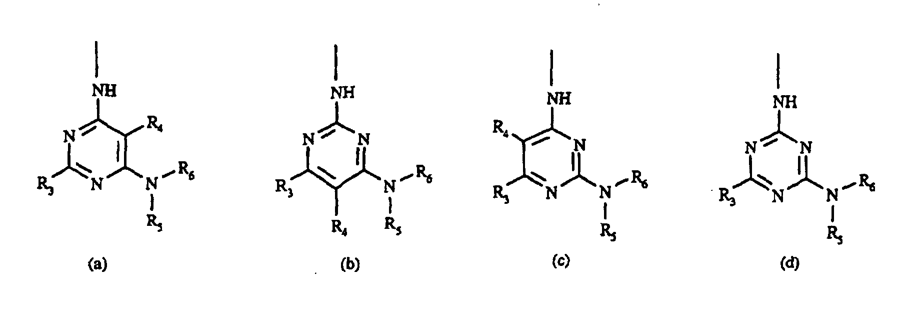 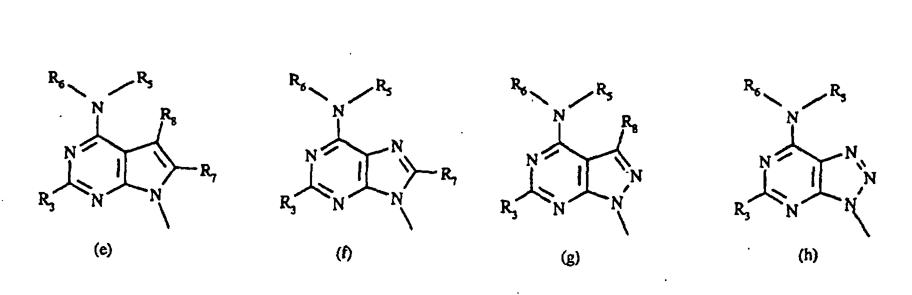 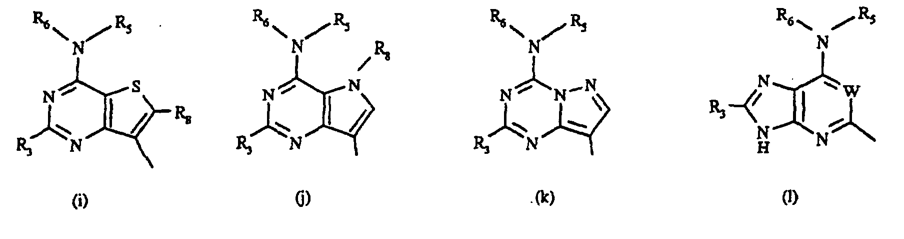 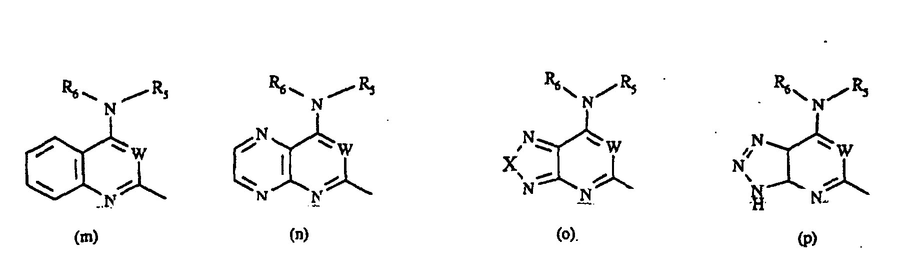
wherein
- R₃ and R₈,: independently, are hydrogen or (C₁₋₄)alkyl,
- R₄: is hydrogen, halogen, (C₁₋₄)alkyl, cyano, nitro, formyl or (C₁₋₄)alkylcarbonyl,
- R₅ and R₆,: independently, are hydrogen, (C₁₋₇)alkyl, (C₃₋₇)alkenyl, (C₃₋₇)cycloalkyl, (C₃₋₇)cycloalkyl (C₁₋₄)alkyl, (C₁₋₄)alkoxy(C₂₋₅)alkyl or benzyl,
- R₇: is hydrogen, hydroxy, (C₁₋₄)alkyl or (C₁₋₄)alkoxy,
- W: is N, C-CN, C-NO₂, C-COH or C-CO-Alk where Alk is as defined above, and
- X: is as defined above,
in free base or acid addition salt form.

GB-A-2 039 450 discloses structurally related compounds lacking the aromatic substituent defined by Het in the compounds of the present application. It has now surprisingly been found that said substituent confers to the compounds antagonistic activity at CRF receptors.

Halogen is fluorine, chlorine, bromine or iodine, preferably fluorine or chlorine.

The radical Het is preferably located on a carbon atom which is adjacent to the heterocyclic moiety in formula I. Preferably Het is of formula (a) to (k), particularly of formula (a).

In a further aspect, the invention provides a process for the production of the compounds of formula I and their salts, whereby a compound of formula II 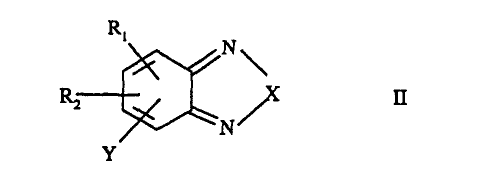 wherein X, R₁ and R₂ are as defined above and Y is a radical having one of the formulae (a') to (p') below: 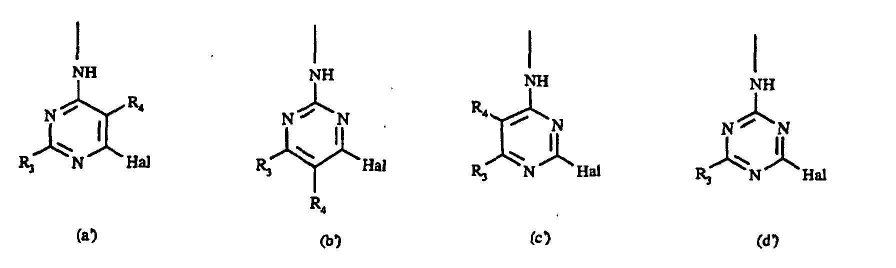 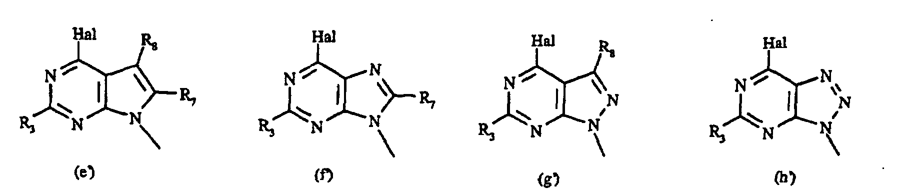 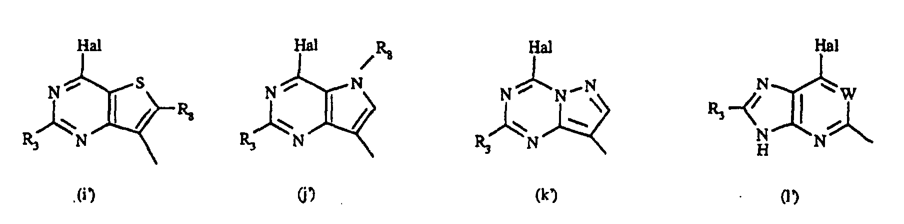 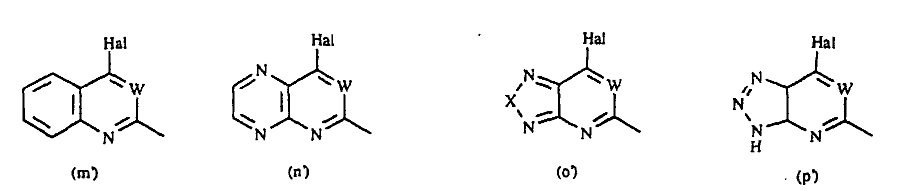 wherein R₃ to R₈, W and X are as defined above and Hal is halogen, is reacted with a compound of formula III 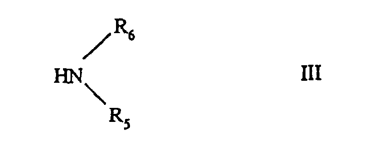 wherein R₅ and R₆ are as defined above, and the resulting compound is recovered in free base form or in acid addition salt form.

The reaction may be effected in known manner, e.g. as described in Example 1. Hal is preferably chlorine, bromine or iodine, particularly chlorine.

Working up of the reaction mixtures obtained according to the above process and purification of the compounds thus obtained may be carried out in accordance to known procedures.

Acid addition salts may be produced in known manner from the free base forms and vice-versa. Suitable pharmaceutically acceptable acid addition salts for use in accordance with the present invention include for example the hydrochloride, the hydrogen maleate, the hydrogen fumarate and the hydrogen malonate.

### The starting materials of formula II may be obtained as follows:

Compounds of formula II wherein Y is a radical having one of the formulae (a') and (d'), may be obtained by reacting a compound of formula IV 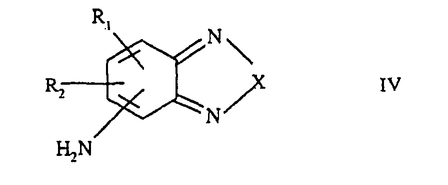 wherein X, R₁ and R₂ are as defined above, with a compound of formula Va or Vd 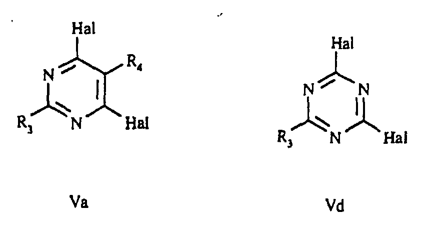 wherein R₃ and R₄ are as defined above and the Hal independently are halogen.

Compounds of formula II wherein Y is a radical having one of the formulae (b'), (c') and (e') to (I') may be obtained by reaction of POCl₃ with a compound of formula VI 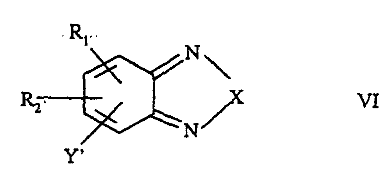 wherein Y' is a radical having one of the formulae (b"), (c") and (e") to (l") below 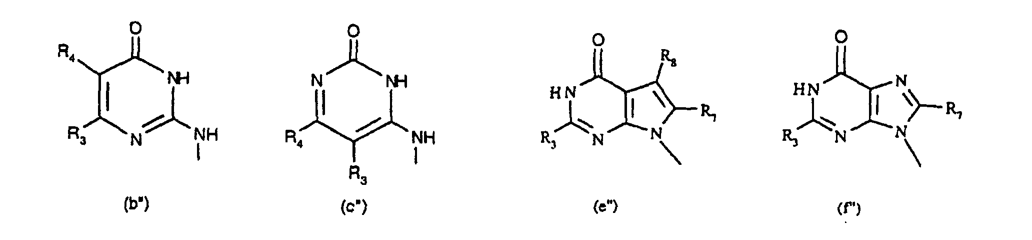 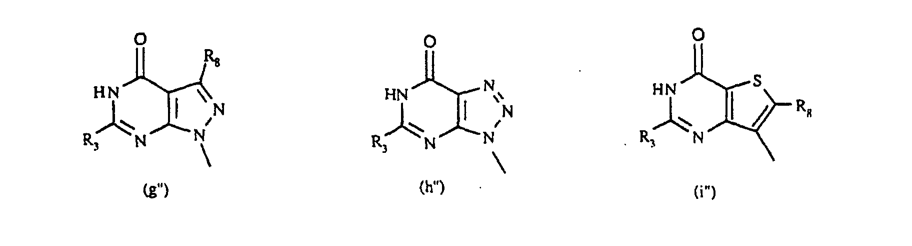 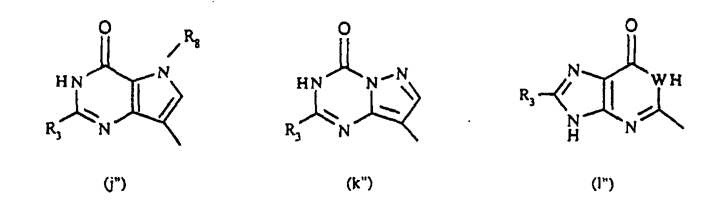 wherein R₃, R₄, R₇, R₈ and W are as defined above.

All the above mentioned reactions are conventional.

The compounds of formulae III, IV, Va, Vd and VI are know or may be obtained from known compounds, using conventional procedures.

Compounds of formula I and their pharmaceutically acceptable acid addition salts, hereinafter referred to as agents of the invention, exhibit valuable pharmacological properties when tested in vitro using corticotropine releasing factor (CRF) receptor expressing cell cultures, and in animals, and are therefore useful as pharmaceuticals.

In particular the agents of the invention bind to CRF receptors. More particularly they exhibit antagonistic activity at CRF₁ receptors, as determined in vitro in the following assay:

Chinese hamster ovary (CHO) cells expressing human recombinant CRF₁ (Chen et al., Proc Natl Acad Sci USA 90, 8967-8971, 1993) are propagated in Dulbecco's modified Eagle medium supplemented with 10% foetal calf serum, non-essential aminoacids, 100U/ml penicillin, 100 mg/1 streptomycin and 1 g/1 geneticin (G418). For cyclic AMP determinations, cells are grown to confluence in 24-multiwell plates. Stimulation of cyclic AMP accumulation by CRF (human/rat form) is measured in intact cells, using the [³H]adenine labelling technique, as described previously (Schoeffter et al., Neuropharmacology 36, 429-437, 1997). Concentration-response curves for CRF are constructed in the presence of putative antagonists (10 µM) or vehicle (dimethyl sulfoxide 1% vol). K_{B} values are calculated from the rightward shifts of the control curve, according to the formula: K_{B} = [antagonist, M]/concentration-ratio-1), where the concentration-ratio designates the ratio of CRF EC₅₀ value in the presence/CRF EC₅₀ value in the absence, of antagonist [Furchgott, In: catecholamines (edited by Blaschko H and Muscholl E) pp. 283-335. Springer, Berlin, 1972].

In this test, the agents of the invention show CRF₁ antagonistic activity with Kb CRF₁ values of about 1 to 500 nM.

The agents according to the invention are therefore useful in the treatment of any state with increased endogenous level of CRF or in which the HPA (hypothalamic pituitary axis) is disregulated, or of various diseases induced or facilitated by CRF, including inflammatory disorders, such as arthritis, asthma and allergies; anxiety including generalized anxiety; phobic and panic attacks; depression; fatigue syndrome; headache; pain, e.g. inflammatory or neuropathic pain; cancer; irritable bowel syndrome, including Crohn's disease, spastic colon and irritable colon; immune dysfunction; human immunodeficiency virus (HIV) infections; neurodegenerative diseases such as senile dementia, Alzheimer's disease and Parkinson's disease; stroke and head trauma; epilepsy; gastrointestinal diseases; eating and body weight disorders such as obesity and anorexia nervosa; hemorrhagic stress; drug and alcohol withdrawal symptoms; drug addiction; sleeping disorders; hormonal disregulations; skin disorders; stress-induced psychotic episodes; fertility problems; sexual dysfunctions and pre-term birth.

The utility of the agents of the invention in the above indicated diseases could be confirmed in a range of standard tests:

For example the anxiolytic activity of the agents of the invention could be confirmed in the mouse elevated plus-maze [see for example Rodgers R.J., Behavioural Pharmacology 8: 477-496 (1997) where the relevance of the elevated plus-maze is discussed on p. 486; for the method, see Rodgers R.J. et al. Ethology and Psychopharmacology (Eds SJ Cooper and CA Hendrie), pp 9-44 (1994), J. Wiley, Chichester]. In this test, the agents of the invention show anxiolytic-like effects on administration of 0.1 to 30 mg/kg p.o.

For the above-mentioned indications, the appropriate dosage will of course vary depending upon, for example, the compound employed, the host, the mode of administration and the nature and severity of the condition being treated. However, in general, satisfactory results in animals are indicated to be obtained at a daily dosage of from about 0.1 to about 100, preferably from about 0.5 to about 100 mg/kg animal body weight. In larger mammals, for example humans, an indicated daily dosage is in the range from yout 1 to about 500, preferably from about 1 to about 300 mg of an agent of the invention, conveniently administered, for example, in divided doses up to four times a day or in sustained release form.

The agent of the invention may be administered by any conventional route, in particular enterally, preferably orally, for example in the form of tablets or capsules, or parenterally, for example in the form of injectable solutions or suspensions.

For the above-mentioned indications a preferred compound is the compound of Example 1 below. In the above-described binding test said compound exhibits CRF₁ antagonistic activity with a Kb CRF₁ of 36 nM. In the above-described elevated plus-maze, doses of 0.1 to 10 mg/kg p.o. (with a maximum at 3mg/kg) significantly affect the anxiety-related behavioural parameters. In contrast to the standard chlordiazepoxide, parameters related to motor stimulation are not affected, which indicates that the observed anxiolytic effects are not attributable to a general motor stimulation.

In accordance with the foregoing, the present invention also provides an agent of the invention, for use as a pharmaceutical, e.g. for the treatment of diseases induced or facilitated by CRF, such as these indicated above.

The present invention furthermore provides a pharmaceutical composition comprising an agent of the invention in association with at least one pharmaceutical carrier or diluent. Such compositions may be manufactured in conventional manner. Unit dosage forms contain, for example, from about 0.25 to about 150, preferably from 0.25 to about 25 mg of a compound according to the invention.

Moreover the present invention provides the use of an agent of the invention, for the manufacture of a medicament for the treatment of any condition mentioned above.

The following examples illustrate the invention. The temperatures are given in degrees Celsius and are uncorrected.

### Example 1: 5,7-dimethyl-4-[2,5-dimethyl-6-(di-n-propyl)-amino-pyrimidin-4-yl]-amino-2,1,3-benzothiadiazole

A solution of 4-(4-chloro-2,5-dimethyl-pyrimidin-6-yl)-amino-5,7-dimethyl-2,1,3-benzothiadiazole (3.5 g) and di-n-propylamine (5.35 ml) in abs. n-methylpyrrolidone (35 ml) is stirred at 157° in a sealed vessel for 96 hours. The reaction is monitored using thin layer chromatography. The reaction mixture is cooled, 50 ml water are added and the aqueous phase is extracted twice with methyl-t-butylether (2x200 ml). The organic phase is dried, evaporated and chromatographically separated on silicagel using cyclohexane/methyl-t-butylethan (5:1). The appropriate fraction is evaporated and the residue recrystallised from methanol to give the title product. Mp =117-119°.

The starting material 4-(4-chloro-2,5-dimethyl-pyrimidin-6-yl)-amino-5,7-dimethyl-2,1,3-benzothiadiazole is produced as follows:
4,6-Dimethyl-2,1,3-benzothiadiazole (7.7 g) is disolved in conc. sulphuric acid (20 ml), cooled under stirring to 0-5° and nitric acid (2.5 ml; d = 1.52) is added dropwise. The clear solution is poured on ice, the so obtained precipitate is filtered off and washed with water. The resulting 5,7-dimethyl-4-nitro-2,1,3-benzothiadiazole is recrystallized from cyclohexane. Mp = 105-106°.

5,7-Dimethyl-4-nitro-2,1,3-benzothiadiazole (20 g) is warmed to ebullition in water (2.2 1) and ethanol (2.2 1) and sodium dithionite is added portionwise (strongly exothermic reaction). The reaction mixture is immediately cooled in an ice bath and extracted with ethyl acetate. The organic phase is concentrated by evaporation and the residue recrystallised in water to give 4-amino-5,7-dimethyl-2,1,3-benzothiadiazole. Mp =113-114°.

4-Amino-5,7-dimethyl-2,1,3-benzothiadiazole (4 g) is added in portions to a 55%-dispersion of sodium hydride (2.72 g) in abs. tetrahydrofuran (50 ml) under an argon atmosphere. The mixture is stirred at 25-30° for 3 hours, then a solution os 4,5-dichloro-2,5-dimethyl-pyrimidine (4 g) in abs. tetrahydrofuran (20 ml) is added in drops at 5° during 30 minutes. The reaction mixture is stirred at room temperature for 16 further hours, then ice water is carefully added. The resulting precipitate is washed with water and a few methanol. After recrystallisation from methanol or cyclohexane, 4-(4-chloro-2,5-dimethyl-pyrimidin-6-yl)-amino-5,7-dimethyl-2,1,3-benzothiadiazole is obtained. Mp = 174-177°.

The following compounds of formula I are prepared analogously to example 1:
a) Compounds of formula

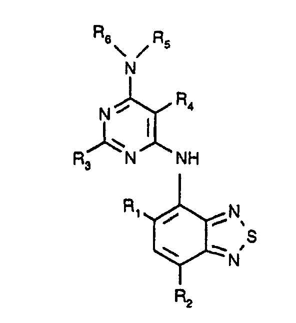

| **Ex.** | **R**_{**1**} | **R**_{**2**} | **R**_{**3**} | **R**_{**4**} | **R**_{**5**} | **R**_{**6**} | **Mp** |
|---|---|---|---|---|---|---|---|
| 2 | Me | Me | Me | Me | n-Propyl | -CH₂-Cyclopropyl | 120-121° |
| 3 | Me | Me | Me | H | n-Propyl | -CH₂-Cyclopropyl | 124-127° * |
| 4 | Me | Me | Me | H | n-Propyl | n-Propyl | 177-179° * |
| 5 | Me | Me | Me | Cl | n-Propyl | n-Propyl | 106-108° |
| 6 | Me | Me | Me | Cl | n-Propyl | -CH₂-Cyclopropyl | 111-113° |
| 7 | Me | Me | Me | Me | n-Propyl | Et | 101-102° |
| 8 | Me | Me | Me | Me | n-Butyl | Et | 83-84° |
| 9 | Me | Me | Me | Me | -CH₂CH₂OCH₃ | -CH₂CH₂OCH₃ | 70-73° |
| 10 | Me | Me | Me | Me | -CH₂-CH(CH₃)-CH₃ | -CH₂-CH(CH₃)-CH₃ | 104-105° |
| 11 | Me | Me | Me | Me | 3-Pentyl | H | 113-115° |
| 12 | Me | Me | Me | Me | 2-Butyl | H | 88° |
| 13 | Me | Me | Me | CN | n-Propyl | n-Propyl | 171-173° |
| 14 | Me | Me | Me | CN | n-Propyl | -CH₂-Cyclopropyl | 165-167° |
| 15 | Cl | Cl | Me | Me | Me | Et | 116° |
| 16 | Cl | Cl | Me | Me | Me | n-Butyl | 105° |
| 17 | Cl | Cl | Me | Me | Et | Et | 189-193° * |
| 18 | Cl | Cl | Me | Me | Et | n-Propyl | 107° |
| 19 | Cl | Cl | Me | Me | Et | n-Butyl | 101° |
| 20 | Cl | Cl | Me | Me | n-Propyl | n-Propyl | 118-121° |
| 21 | Cl | Cl | Me | Me | n-Propyl | -CH₂-Cyclopropyl | 120-123° |
| 22 | Cl | Cl | Me | Me | n-Butyl | n-Butyl | 56° |
| 23 | Cl | Cl | Me | Me | Allyl | Allyl | 112° |
| 24 | Cl | Cl | Me | Me | H | Benzyl | 126° |
| 25 | Cl | Cl | Me | Me | Me | Benzyl | 138° |
| 26 | Me | Cl | Me | Me | n-Propyl | n-Propyl | 102-104° |
| 27 | Me | Cl | Me | Me | n-Propyl | -CH₂-Cyclopropyl | 131-132° |
| 28 | Cl | Me | Me | Me | n-Propyl | -CH₂-Cyclopropyl | 110-112° |
| 29 | Cl | Me | Me | Me | n-Propyl | n-Propyl | 112-114° |
| 30 | Cl | H | Me | Me | n-Propyl | n-Propyl | 77-80° |
| 31 | Me | H | Me | Me | n-Propyl | n-Propyl | 95-98° |

| | | | | | | | |
|---|---|---|---|---|---|---|---|
| Me = Methyl; Et = ethyl; *: Fumarate | | | | | | | |

b) Compounds of formula

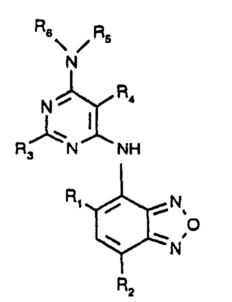

| | | | | | | | |
|---|---|---|---|---|---|---|---|
| **Ex.** | **R**_{**1**} | **R**_{**2**} | **R**_{**3**} | **R**_{**4**} | **R**_{**5**} | **R**_{**6**} | **Mp** |
| 32 | Me | Me | Me | Me | n-Propyl | n-Propyl | 123° |
| 33 | Cl | Me | Me | Me | n-Propyl | n-Propyl | 100° |
| 34 | Me | Me | Me | Me | n-Propyl | -CH₂-Cyclopropyl | 105° |
| 35 | Cl | Me | Me | Me | n-Propyl | -CH₂-Cyclopropyl | 86° |
| 36 | Me | Me | Me | Me | n-Propyl | Et | 102° |
| 37 | Me | Me | Me | Me | n-Butyl | Et | 71° |
| Me = Methyl; Et = Ethyl | | | | | | | |

c) Compounds of formula

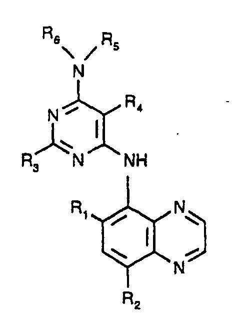

| | | | | | | | |
|---|---|---|---|---|---|---|---|
| **Ex.** | **R**_{**1**} | **R**_{**2**} | **R**_{**3**} | **R**_{**4**} | **R**_{**5**} | **R**_{**6**} | **Mp** |
| 38 | Me | Me | Me | Me | n-Propyl | n-Propyl | 94-96° |
| 39 | Cl | Cl | Me | Me | n-Propyl | n-Propyl | 126-128° |
| 40 | Cl | Me | Me | Me | n-Propyl | n-Propyl | 107-109° |
| 41 | Cl | Me | Me | Me | n-Propyl | -CH₂-Cyclopropyl | 115-117° |
| 42 | Me | Me | Me | Me | n-Propyl | -CH₂-Cyclopropyl | 105-106° |
| 43 | Cl | Cl | Me | Me | n-Propyl | -CH₂-Cyclopropyl | 126-127° |
| 44 | Cl | Cl | Me | Me | Et | n-Propyl | 120-123° |
| 45 | Me | Me | Me | Me | Et | n-Propyl | 109-110° |
| 46 | Cl | Cl | Me | Me | Et | n-Butyl | 117-119° |
| 47 | Me | Me | Me | Me | Et | n-Butyl | 106-107° |
| Me = Methyl; Et = Ethyl | | | | | | | |

d) Compounds of formula

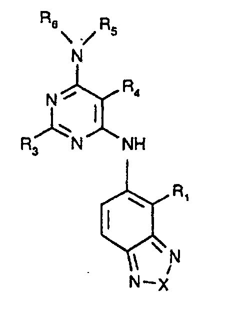

| | | | | | | | |
|---|---|---|---|---|---|---|---|
| **Ex.** | **X** | **R**_{**1**} | **R**_{**3**} | **R**_{**4**} | **R**_{**5**} | **R**_{**6**} | **Mp** |
| 48 | S | Cl | Me | Me | Et | n-Butyl | 85° |
| 49 | S | Cl | Me | Me | n-Propyl | -CH₂-Cyclopropyl | 84° |
| 50 | CH=CH | Br | Me | Me | Et | n-Butyl | 110° |
| 51 | CH=CH | Br | Me | Me | n-Propyl | -CH₂-Cyclopropyl | 135° |
| Me = Methyl; Et = Ethyl | | | | | | | |

e) Compounds of formula

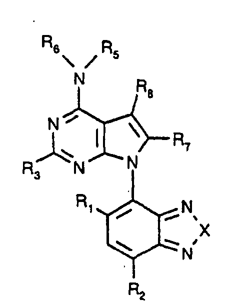

| | | | | | | | | | |
|---|---|---|---|---|---|---|---|---|---|
| **Ex.** | **X** | **R**_{**1**} | **R**_{**2**} | **R**_{**3**} | **R**_{**5**} | **R**_{**6**} | **R**_{**7**} | **R**_{**8**} | **Mp** |
| 52 | S | Me | Me | Me | Et | n-Butyl | Me | Me | 90-92° |
| 53 | CH=CH | Me | Me | Me | Et | n-Butyl | Me | Me | 106-108° |
| 54 | S | Me | Me | Me | n-Propyl | n-Propyl | Me | Me | 126-128° |
| 55 | CH=CH | Me | Me | Me | n-Propyl | n-Propyl | Me | Me | 156-158° |
| 56 | S | Cl | Me | Me | n-Propyl | n-Propyl | Me | Me | 135-136° |
| 57 | CH=CH | Cl | Me | Me | n-Propyl | n-Propyl | Me | Me | 130-131° |
| 58 | S | Cl | Me | Me | Et | n-Butyl | Me | Me | 90-92° |
| 59 | CH=CH | Cl | Me | Me | Et | n-Butyl | Me | Me | 177-178° |
| Me = Methyl; Et = Ethyl | | | | | | | | | |

f) The compound of formula

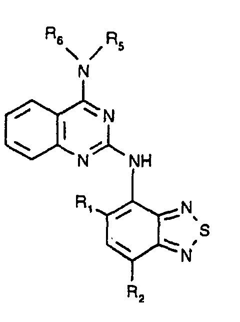

| **Ex.** | **R**_{**1**} | **R**_{**2**} | **R**_{**5**} | **R**_{**6**} | **Mp** |
|---|---|---|---|---|---|
| 60 | Me | Me | n-Propyl | -CH₂-Cyclopropyl | 234-237°* |

| | | | | | |
|---|---|---|---|---|---|
| Me = Methyl; *: Fumarate | | | | | |

## Claims

1. A compound of formula I 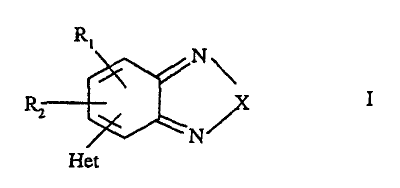 wherein
X is O, S, N-CH₃, CH=CH or CAlk = CAlk, where the Alk independently are (C₁₋₄)alkyl,
R₁ and R₂ independently, are hydrogen, halogen, (C₁₋₄)alkyl, (C₁₋₄)alkoxy or trifluoromethyl, and
Het is a radical having one of the formulae (a) to (p) below: 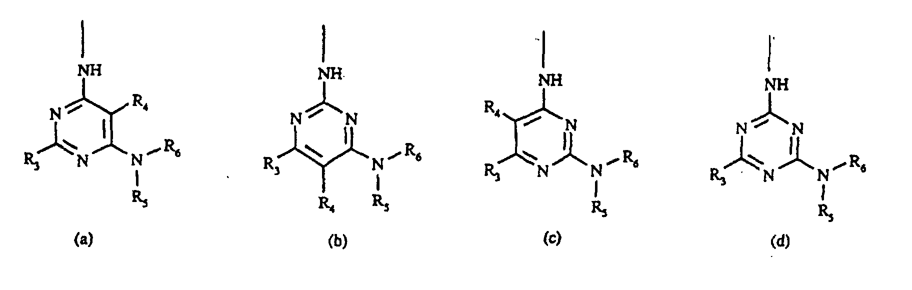 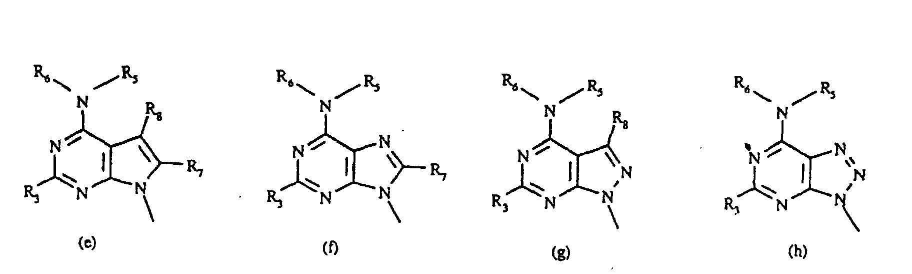 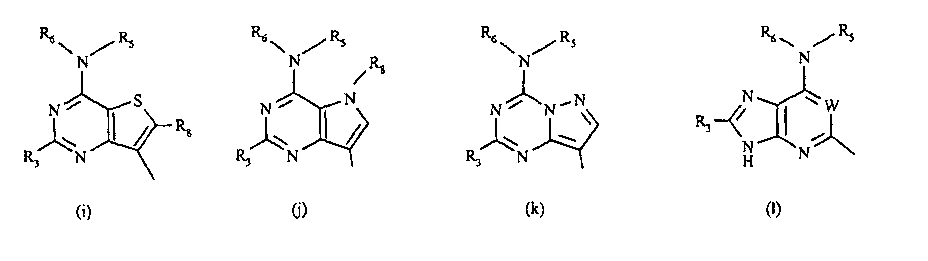 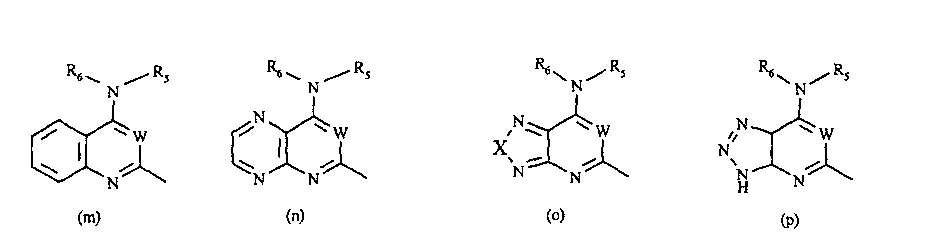
wherein
R₃ and R₈, independently, are hydrogen or (C₁₋₄)alkyl,
R₄ is hydrogen, (C₁₋₄)alkyl, cyano, nitro, formyl or (C₁₋₄)alkylcarbonyl,
R₅ and R₆, independently, are hydrogen, (C₁₋₇)alkyl, (C₃₋₇)alkenyl, (C₃₋₇)cycloalkyl, (C₃₋₇)cycloalkyl (C₁₋₄)alkyl, (C₁₋₄)alkoxy(C₂₋₅)alkyl or benzyl,
R₇ is hydrogen, hydroxy, (C₁₋₄)alkyl or (C₁₋₄)alkoxy,
W is N, C-CN, C-NO₂, C-COH or C-CO-Alk where Alk is as defined above, and
X is as defined above,
in free base or acid addition salt form.

2. 5,7-Dimethyl-4-[2,5-dimethyl-6-(di-n-propyl)-amino-pyrimidin-4-yl]amino-2,1,3-benzothiadiazole in free base or acid addition salt form.

3. N-(6-Chloro-8-methyl-quinoxalin-5-yl)-N'-cyclopropylmethyl-2,5-dimethyl-N'-propylpyrimidine-4,6-diamine in free base or acid addition salt form.

4. A process for the preparation of a compound of formula I as defined in claim 1, or a salt thereof, which includes the step of reacting a compound of formula II 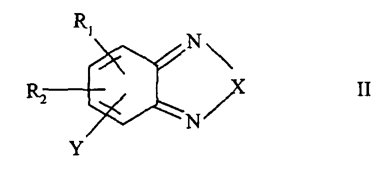 wherein X, R₁ and R₂ are as defined in claim 1 and Y is a radical having one of the formulae (a') to (p') below: 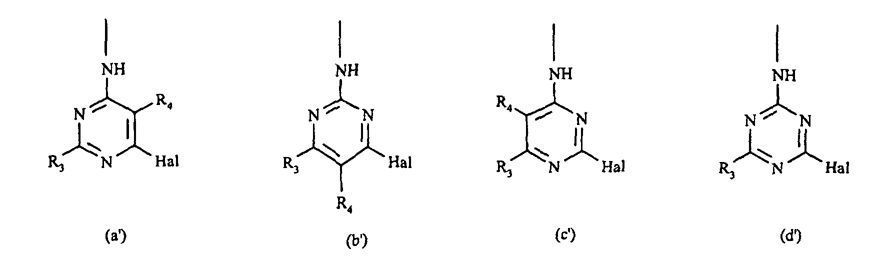 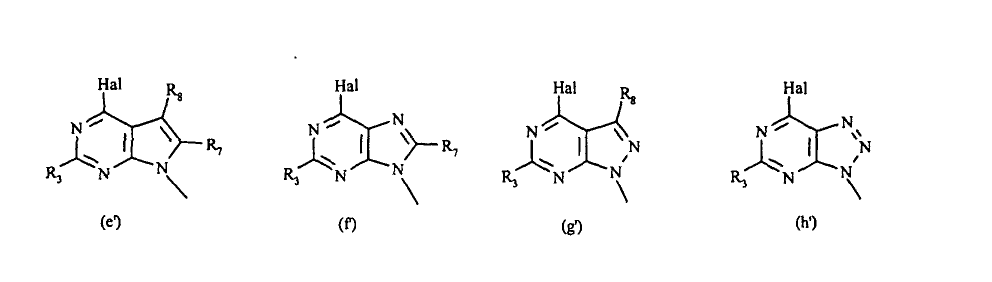 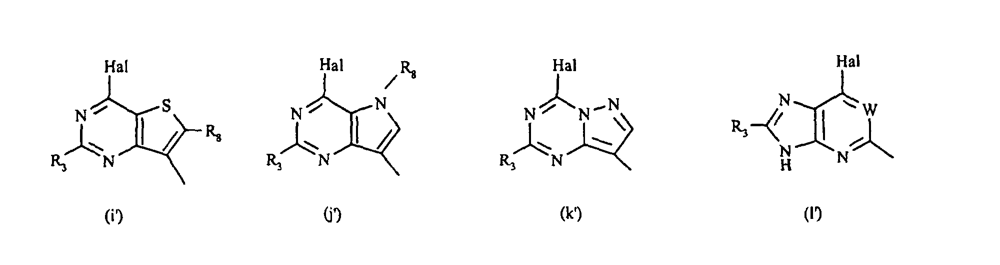 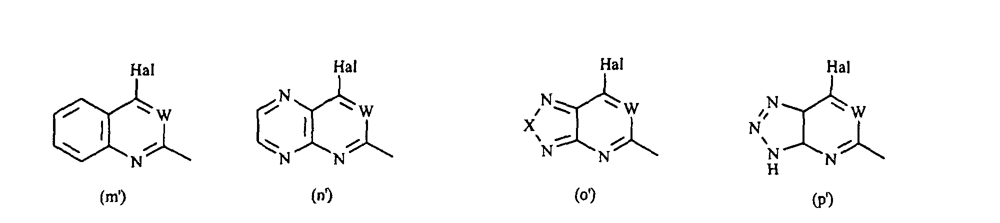 wherein R₃ to R₈, W and X are as defined in claim 1 and Hal is halogen, with a compound of formula III 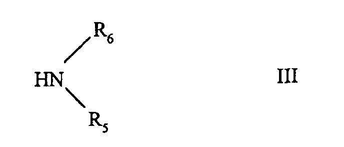 wherein R₅ and R₆ are as defined in claim 1, and recovering the thus obtained compound of formula I in free base or acid addition salt form.

5. A compound of any of claims 1 to 3 in free base or pharmaceutically acceptable acid addition salt form , for use as a pharmaceutical.

6. A compound of any of claims I to 3 in free base or pharmaceutically acceptable acid addition salt form, for use in the treatment of any state with increased endogenous level of CRF or in which the HPA is disregulated, or of a disease induced or faciliated by CRF.

7. A pharmaceutical composition comprising a compound of any of claims 1 to 3 in free base or pharmaceutically acceptable acid addition salt form, in association with a pharmaceutical carrier or diluent.

8. A compound of any of claims 1 to 3 in free base or pharmaceutically acceptable acid addition salt form, for use as a pharmaceutical for the treatment of any state with increased endogenous level of CRF or in which the HPA is disregulated, or of a disease induced or facilitated by CRF.

9. The use of a compound of any of claims 1 to 3 in free base or pharmaceutically acceptable acid addition salt form, for the manufacture of a medicament for the treatment of any state with increased endogenous level of CRF or in which the HPA is disregulated, or of a disease induced or facilitated by CRF.

## Patentansprüche

1. Verbindung der Formel I 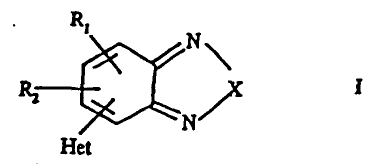 worin
X für O, S, N-CH₃, CH=CH oder CAIk=CAtk steht, worin Alk unabhängig für C₁-C₄ Alkyl steht,
R₁ und R₂ unabhängig für Wasserstoff, Halogen, C₁-C₄ Alkyl, C₁-C₄ Alkoxy oder Trifluormethyl stehen, und
Het für einen Rest mit einer der folgenden Formeln (a) bis (p) steht 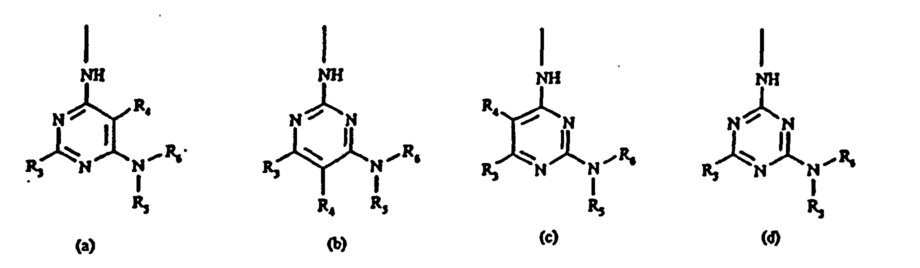 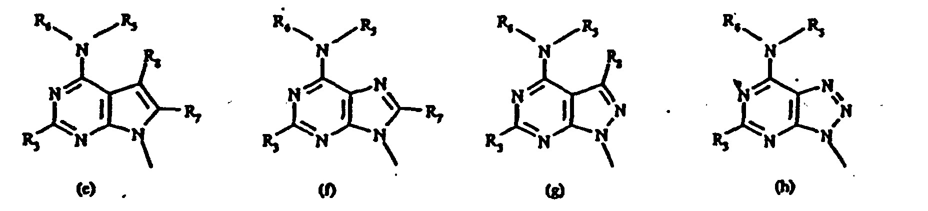 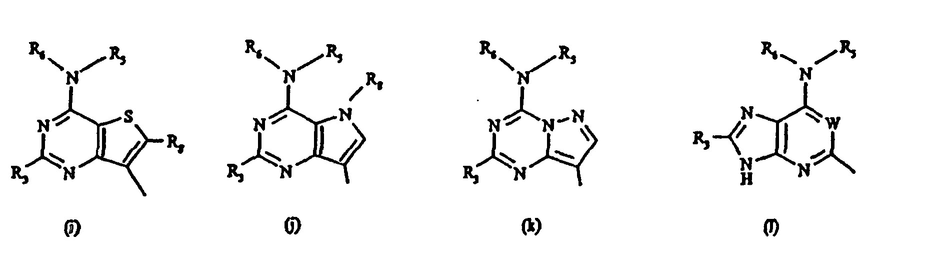 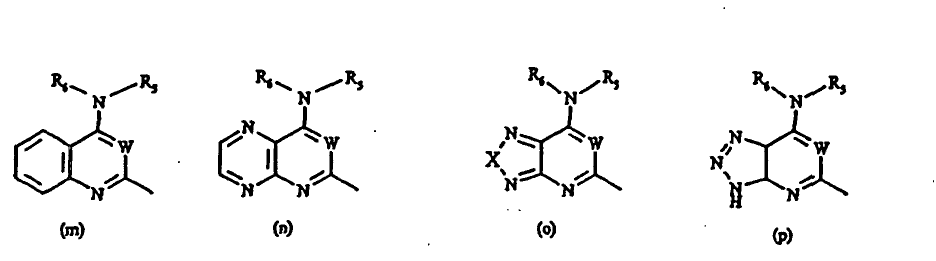
worin
R₃ und R₈ unabhängig für Wasserstoff oder C₁-C₄ Alkyl stehen,
R₄ für Wasserstoff, C₁-C₄ Alkyl, Cyano, Nitro, Formyl oder C₁-C₄ Alkylcarbonyl steht,
R₅ und R₆ unabhängig für Wasserstoff, C₁-C₇ Alkyl, C₃-C₇ Alkenyl, C₃-C₇ Cycloalkyl, C₃-C₇-Cycloalkyl-C₁-C₄-alkyl, C₁-C₄ Alkoxy-C₂-C₅-alkyl oder Benzyl stehen,
R₇ für Wasserstoff, Hydroxy, C₁-C₄ Alkyl oder C₁-C₄ Alkoxy steht,
W für N, C-CN, C-NO₂, C-COH oder C-CO-Alk steht, worin Alk wie oben definiert ist, und
X wie oben definiert ist,
in Form der freien Base oder in Säureadditionssalzform

2. 5,7-Dimethyl-4-[2,5-dimethyl-6-(di-n-propyl)aminopyrimidin-4-yl]amino-2,1,3-benzothiadiazol in Form der freien Base oder in Säureadditionssalzform.

3. N-(6-Chlor-8-methylchinoxalin-5-yl)-N'-cyclopropylmethyl-2,5-dimethyl-N'-propylpyrimidin-4,6-diamin in Form der freien Base oder in Säureadditionssalzform.

4. Verfahren zur Herstellung einer Verbindung der Formel I nach Anspruch 1 oder eines Salzes hiervon, das den Schritt der Umsetzung einer Verbindung der Formel II 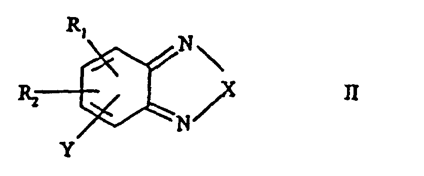 worin X, R₁ und R₂ wie in Anspruch 1 definiert sind und Y für einen Rest mit einer der folgenden Formeln (a') bis (p') steht 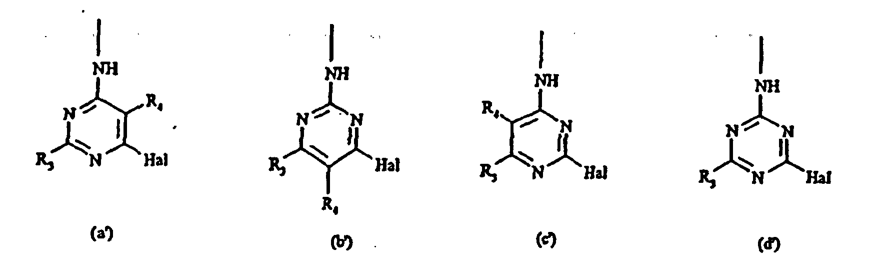 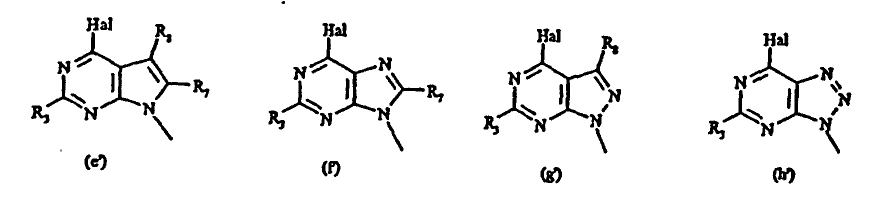 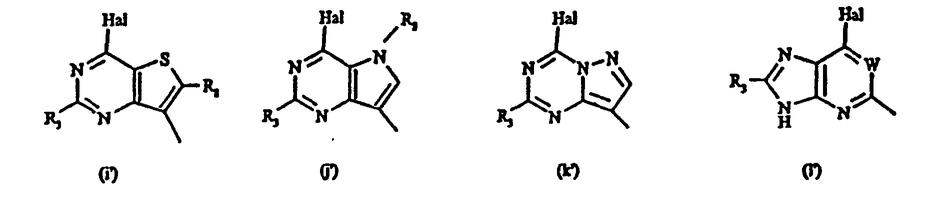 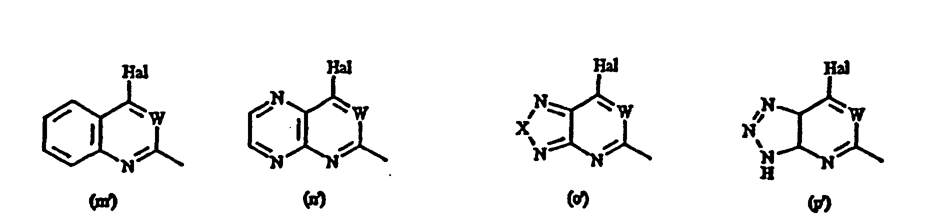 worin R₃ bis R₈, W und X wie in Anspruch 1 definiert sind und Hai fiir Halogen steht, mit einer Verbindung der Formel III umfaßt 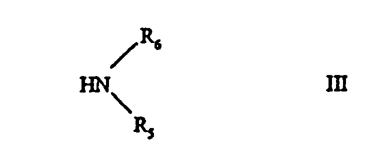 worin R₅ und R₆ wie in Anspruch 1 definiert sind, und Gewinnen der so erhaltenen Verbindung der Formel I in Form der freien Base oder in Säureadditionssalzform.

5. Verbindung nach einem der Ansprüche 1 bis 3 in Form der freien Base oder in pharmazeutisch annehmbarer Säureadditionssalzform zur Verwendung als Pharmazeutikum.

6. Verbindung nach einem der Ansprüche 1 bis 3 in Form der freien Base oder in pharmazeutisch annehmbarer Säureadditionssalzform zur Verwendung bei der Behandlung jedes Zustands mit erhöhtem endogenem Spiegel an CRF oder worin das HPA dereguliert ist, oder einer Erkrankung, die durch CRF hervorgerufen oder gefördert wird.

7. Pharmazeutische Zusammensetzung, die eine Verbindung nach einem der Ansprüche 1 bis 3 in Form der freien Base oder in pharmazeutisch annehmbarer Säureadditionssalzform zusammen mit einem pharmazeutisch annehmbaren Träger oder Verdünnungsmittel enthält.

8. Verbindung nach einem der Ansprüche 1 bis 3 in Form der freien Base oder in pharmazeutisch annehmbarer Säureadditionssalzform zur Verwendung als Pharmazeutikum zur Behandlung jedes Zustands mit erhöhtem endogenem Spiegel an CRF oder worin das HPA dereguliert ist, oder einer Erkrankung, die durch CRF hervorgerufen oder gefördert wird.

9. Verwendung einer Verbindung nach einer der Ansprüche 1 bis 3 in Form der freien Base oder in pharmazeutisch annehmbarer Säureadditionssalzform zur Herstellung eines Arzneimittels zur Behandlung jedes Zustands mit erhöhtem endogenem Spiegel an CRF oder worin das HPA dereguliert ist, oder einer Erkrankung, die durch CRF hervorgerufen oder gefördert wird.

## Revendications

1. Composé de formule I 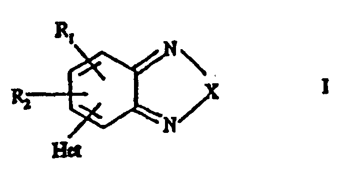 dans laquelle
X représente O, S, N-CH₃, CH=CH ou CAlk=CAlk, où les Alk représentent indépendamment des radicaux alkyle en C₁ à C₄ ;
R₁ et R₂ représentent indépendamment un hydrogène, un halogène, un alkyle en C₁ à C₄, un alcoxy en C₁ à C₄ ou un trifluorométhyle, et
Het est un radical ayant l'une des formules (a) à (p) ci-dessous: 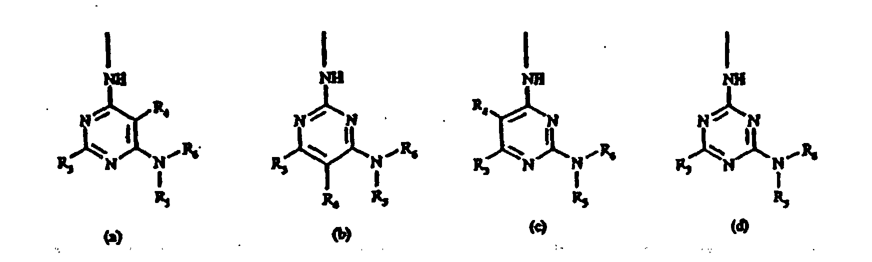 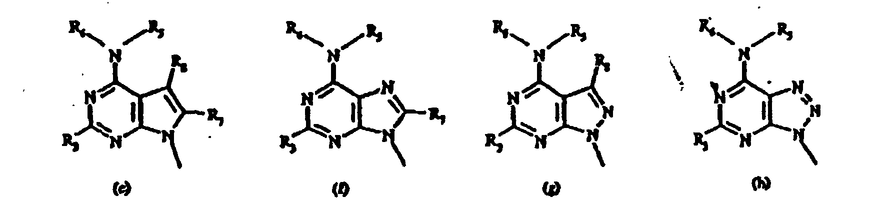 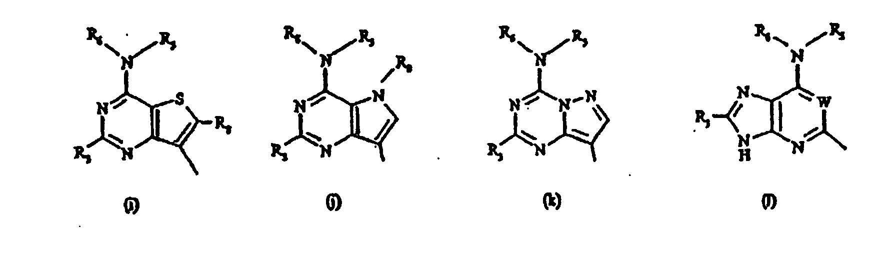 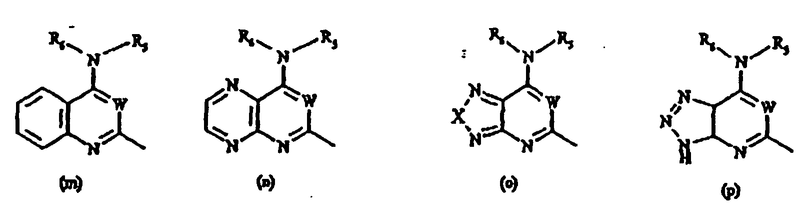 dans lesquelles
R₃ et R₈ représentent, indépendamment, un hydrogène ou un alkyle en C₁ à C₄,
R₄ est un hydrogène, un alkyle en C₁ à C₄, un cyano, un nitro, un formyle ou un alkyle en C₁ à C₄-carbonyle,
R₅ et R₆, indépendamment, représentent un hydrogène, un alkyle en C₁ à C₇, un alcényle en C₃ à C₇, un cycloalkyle en C₃ à C₇, un cycloalkyle en C₃ à C₇-alkyle en C₁ à C₄, un alcoxy en C₁ à C₄-alkyle ou benzyle,
R₇ est un hydrogène, un hydroxy, un alkyle en C₁ à C₄ ou un alcoxy en C₁ à C₄,
W représente N, C-N, C-NO₂, C-COH ou C-CO-Alk où Alk est tel que défini ci-dessus, et
X est tel que défini ci-dessus,
sous forme de base libre ou de sel d'addition d'acide.

2. 5,7-dhnéthyl-4-[2,5-diméthyl-6-(di-n-propyl)-amino-pyrimidin-4-yl]amino-2,1,3-benzothiadiazole sous forme de base libre ou de sel d'addition d'acide.

3. N-(6-chloro-8-méthyl-quinoxalin-5-yl)-N'-cyclopropylméthyl-2,5-diméthyl-N'-propyl-pyrimidine-4,6-diamine sous forme de base libre ou de sel d'addition d'acide.

4. Procédé de préparation d'un composé de formule I telle que définie dans la revendication 1, ou d'un de ses sels, qui inclut l'étape consistant à faire réagir un composé de formule II 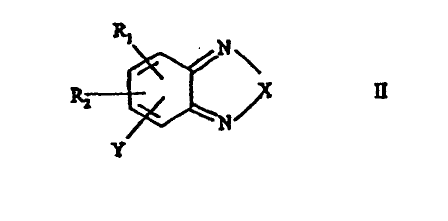 dans laquelle X, R₁ et R₂ sont tels que définis dans la revendication 1 et Y est un radical ayant l'une des formules (a') à (p') ci-dessous ; 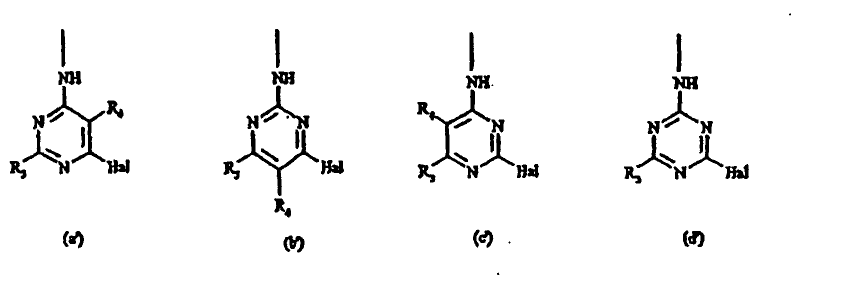 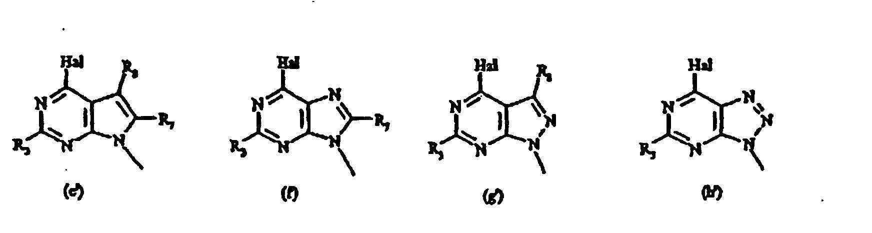 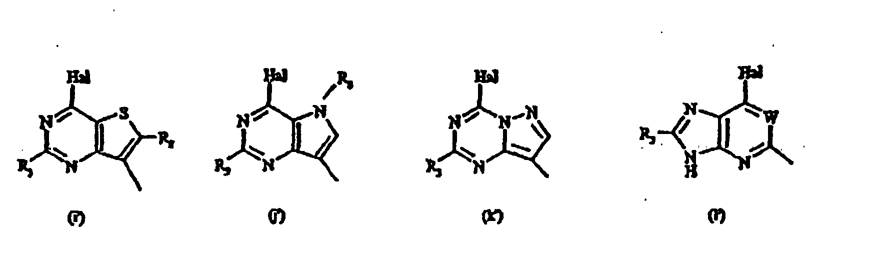 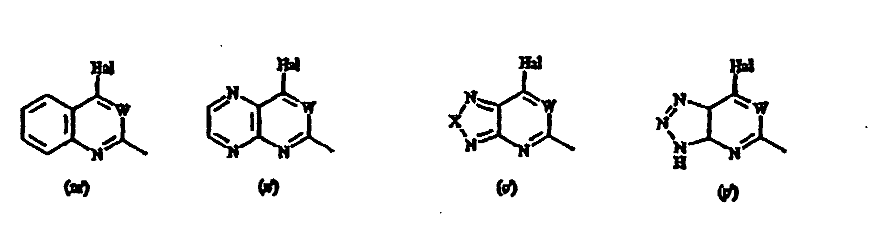 dans lesquelles R₃ à R₈, W et X sont tels que définis dans la revendication 1 et Hal est un halogène, avec un composé de formule III 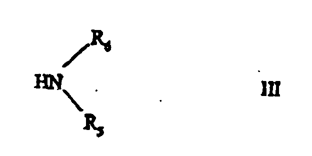 dans laquelle R₅ et R₆ sont tels que définis dans la revendication 1, et à recueillir le composé de formule I ainsi obtenu sous forme de base libre ou de sel d'addition d'acide.

5. Composé de l'une quelconque des revendications 1 à 3 sous forme de base libre ou de sel d'addition d'acide pharmaceutiquement acceptable, pour l'utilisation comme produit pharmaceutique.

6. Composé de l'une quelconque des revendications 1 à 3 sous forme de base libre ou de sel d'addition d'acide pharmaceutiquement acceptable, pour l'utilisation dans le traitement de tout état présentant un niveau accru de CRF (facteur de libération de corticotropine) endogène ou dans lequel l'HPA est dérégulé, ou d'une maladie induite ou facilitée par le CRF.

7. Composition pharmaceutique comprenant un composé de l'une quelconque des revendications 1 à 3 sous forme de base libre ou de sel d'addition d'acide pharmaceutiquement acceptable, en association avec un support ou diluant pharmaceutique.

8. Composé de l'une quelconque des revendications 1 à 3 sous forme de base libre ou de sel d'addition d'acide pharmaceutiquement acceptable, aux fins d'utilisation comme produit pharmaceutique pour le traitement de tout état présentant un niveau accru de CRF endogène ou dans lequel l'HPA est dérégulé, ou d'une maladie induite ou facilitée par le CRF.

9. Utilisation d'un composé de l'une quelconque des revendications 1 à 3 sous forme de base libre ou de sel d'addition d'acide pharmaceutiquement acceptable, en vue de la fabrication d'un médicament pour le traitement de tout état présentant un niveau accru de CRF endogène ou dans lequel l'HPA est dérégulé, ou d'une maladie induite ou facilitée par le CRF.
